# EUROPEAN PATENT APPLICATION

(11) **EP 2 087 921 A1**
(43) Date of publication of application: **12.08.2009**
(21) Application number: 07832076.9
(22) Date of filing: 19.11.2007
(51) Int. Cl.: A61M 39/02, F16L 37/08

(54) **CONNECTOR**

(30) Priority: 24.11.2006 JP 2006317773
(71) Applicant: Terumo Kabushiki Kaisha, Shibuya-ku Tokyo 151-0072 (JP)
(72) Inventor: HISHIKAWA, Yoshinori, Nakakoma-gun Yamanashi 409-3853 (JP); YOKOTA, Takayuki, Nakakoma-gun Yamanashi 409-3853 (JP)
(74) Representative: TBK-Patent
(86) International application number: PCT/JP2007/072346
(87) International publication number: WO 2008/062742

(57) **Abstract**

A connector has a first structure and a second structure. The first structure has a tubular male connector having a male-side cavity and also has a tubular female connector having a female-side cavity communicating with the male-side cavity and allowing another male connector same as the male connector to be connected to the female-side cavity. The second structure has an installation section capable of being installed on the first structure, a male lock section placed, in an installed state where the male lock section is installed on the first structure, adjacent to the male connector such that the direction of connection of the male lock section is parallel to that of the male connector, and a female lock section placed, in the installed state, adjacent to the female connector such that the direction of connection of the female lock section is parallel to that of the female connector, and allowing another male lock section same as the male lock section to be connected to the female lock section. The way of use of the first structure can be selected between use with the second structure installed on it and use without the second structure installed on it.

## Description

### TECHNICAL FIELD

The present invention relates to a connector.

### BACKGROUND ART

In medical appliances requiring connection of passages for liquid(s) to be used for infusion, transfusion, nutrient dosing or the like, the liquid passages (circuits) must be connected and disconnected as required when sustainedly or momentarily causing a flow of the liquid(s), such as a liquid medicine, blood, and liquid food. In such a situation, it is known to attach a connection means for connecting the liquid passages to an intermediate portion of the circuit. Typical examples of the connection means include the one described in Patent Document 1.

The connection means (connector) includes a male connector section, a female connector section, a male lock section, and a female lock section. In the connection means configured in this manner, the male connector section, the female connector section, the male lock section, and the female lock section are coupled to one another or are formed integrally.

In the connection means (described in Patent Document 1) according to the related art, however, there have been cases where, depending on the shape(s) of a male lock section and/or a female lock section on the side thereof that is coupled with the connection means, it is impossible to connect the connection means with the mating body. Where connection is impossible, in medical appliances, it has been a common practice to replace one of the connection means and the mating body in order to enable the intended connection, thereby securing a passage for a liquid.
Patent Document 1: International Publication No. WO 2006/068211

### DISCLOSURE OF INVENTION

It is an object of the present invention to provide a connector rich in versatility, in which the connector can be modified in accordance with a mating body to be connected therewith, so as to enable connection between the connector and the mating body.

In order to attain the above object, the present invention provides a connector including:
a first structure including a tubular male connector section that has a male-side cavity, and a tubular female connector section having a female-side cavity, which communicates with the male-side cavity and to which another male connector section the same as the male connector section can be connected; and
a second structure including an installation section that can be installed on the first structure, a male lock section disposed adjacent to the male connector section in an installed condition in which the second structure is installed on the first structure, so that the direction of connection thereof is parallel to that of the male connector section, and a female lock section to which another male lock section the same as the male lock section can be connected, which is disposed adjacent to the female connector section in the installed condition, so that the direction of connection thereof is parallel to that of the female connector section;
wherein the first structure can be used selectively with either the second structure installed thereon or without the second structure installed thereon.

This ensures that the connector can be modified in accordance with a mating body to be connected thereto, in order to enable connection between the connector and the mating body. Therefore, a connector rich in versatility can be realized.

In addition, in the connector according to the present invention, preferably, the first structure is used with the second structure installed thereon in a case where a mating body using the first structure has the other male connector section and the other male lock section.

This makes it possible to connect the connector to the mating body assuredly.

Further, in the connector according to the present invention, preferably, the female connector section is formed with a screw part on an outer peripheral portion thereof.

This ensures that, in the event that another male connector section has a screw part capable of screw engagement with the screw part of the female connector section, the connector sections can securely be connected to each other.

Further, in the connector according to the present invention, preferably, the male connector section is formed with a columnar part, which has a substantially columnar outside shape and a center axis that is substantially orthogonal to a center axis of an opening part of the male connector section; and
the installation section is composed of a recess, which is formed in the vicinity of the male lock section and into which the columnar part can be fitted, and a small plate-like piece having a hole through which the male connector section can pass.

This ensures that the first structure is fixed to the second structure through the installation section.

In addition, in the connector according to the present invention, preferably, the installation section is composed of a ring-shaped section, into which the outer peripheral portion of the female connector section can be fitted.

This ensures that the first structure is fixed to the second structure through the installation section.

Further, in the connector according to the present invention, preferably, the male lock section is provided with a male-side engaging part, and includes a pair of claw parts, which can move toward and away from each other, and an urging part for urging both of the claw parts so as to move the claw parts away from each other; and
the female lock section includes a female-side engaging part capable of engagement with a male-side engaging part of another male lock section, which is the same as the male lock section, and an operating part operable to cause both of the claw parts of the male lock section to move closer to each other.

This makes it possible to connect the female lock section and the other male lock section to each other assuredly, as well as to assuredly release the connected condition.

Further, in the connector according to the present invention, preferably, each of the claw parts projects respectively in the same direction as the male connector section in the installed condition.

This ensures that, at the time of connecting another male lock section to the female lock section, the connecting operation can be carried out easily.

In addition, in the connector according to the present invention, preferably, each of the claw parts projects in a direction opposite to the male connector section in the installed condition.

This ensures that, at the time of connecting another male lock section to the female lock section, the connecting operation can be performed easily.

Further, in the connector according to the present invention, preferably, the female connector section and the male connector section are arranged such that center lines thereof are parallel to each other, and an opening part of the female connector section and an opening part of the male connector section are oriented in opposite directions.

This ensures that, for example, when the connector and a mating body are connected, they can be arranged substantially rectilinearly.

Further, in the connector according to the present invention, preferably, the female connector section and the male connector section are arranged such that center lines thereof are substantially orthogonal to each other.

This makes it possible to change the direction of flow of liquid that passes through the connector.

In order to attain the above object, the present invention further provides a connector including:
a first structure including a tubular male connector section having a male-side cavity; and
a second structure including an installation section that can be installed on the first structure, and a male lock section disposed adjacent to the male connector section in an installed condition in which the second structure is installed on the first structure, so that the direction of connection thereof is parallel to that of the male connector section;
wherein the first structure can be used selectively with either the second structure installed thereon or without the second structure installed thereon.

This ensures that the connector can be modified in accordance with a mating body to be connected thereto, in order to enable connection between the connector and the mating body. Therefore, a connector rich in versatility can be realized.

Further, in the connector according to the present invention, preferably, the first structure is used without the second structure installed thereon, in a case where the mating body using the first structure has the other male connector section, but does not have the other male lock section.

This makes it possible to connect the connector to the mating body reliably.

Further, in the connector according to the present invention, preferably, the first structure is configured such that, once the second structure has been installed on the first structure, the second structure cannot be detached therefrom.

This ensures that when the connector is used, unintentional disengagement of the second structure from the first structure can be prevented from occurring.

### BRIEF DESCRIPTION OF DRAWINGS

FIG. 1 is an exploded perspective view showing a first embodiment of the connector according to the present invention;
FIG. 2 is a schematic view of the connector shown in FIG. 1, as viewed from the side of the arrow A in FIG. 1;
FIG. 3 is a schematic view of the connector shown in FIG. 1, as viewed from the side of the arrow B in FIG. 1;
FIG. 4 is a perspective view showing an exemplary form in which the connector shown in FIG. 1 is used;
FIG. 5 is a perspective view showing an exemplary form in which the connector shown in FIG. 1 is used;
FIG. 6 shows partial longitudinal sectional views, illustrating a process of connecting the connector (female lock section), when used in the form shown in FIG. 4;
FIG. 7 shows partial longitudinal sectional views illustrating a process of canceling the connection of the connector (female lock section), when used in the form shown in FIG. 4;
FIG. 8 is a perspective view of a valve element possessed by the connector shown in FIG. 1;
FIG. 9 is a longitudinal sectional view of a female connector section of the connector shown in FIG. 1;
FIG. 10 is a longitudinal sectional view showing a connected state of the connector (female connector section), when used in the form shown in FIG. 4;
FIG. 11 is a longitudinal sectional view showing a connected state of the connector (female connector section), when used in the form shown in FIG. 5;
FIG. 12 is an exploded perspective view showing a second embodiment of the connector according to the present invention;
FIG. 13 is an exploded perspective view showing a third embodiment of the connector according to the present invention;
FIG. 14 is an exploded perspective view showing a fourth embodiment of the connector according to the present invention;
FIG. 15 is an exploded perspective view showing a fifth embodiment of the connector according to the present invention; and
FIG. 16 is an exploded perspective view showing a sixth embodiment of the connector according to the present invention.

### BEST MODE FOR CARRYING OUT THE INVENTION

The connector according to the present invention will be described in detail below, based on preferred embodiments thereof shown in the accompanying drawings.

### <First Embodiment>

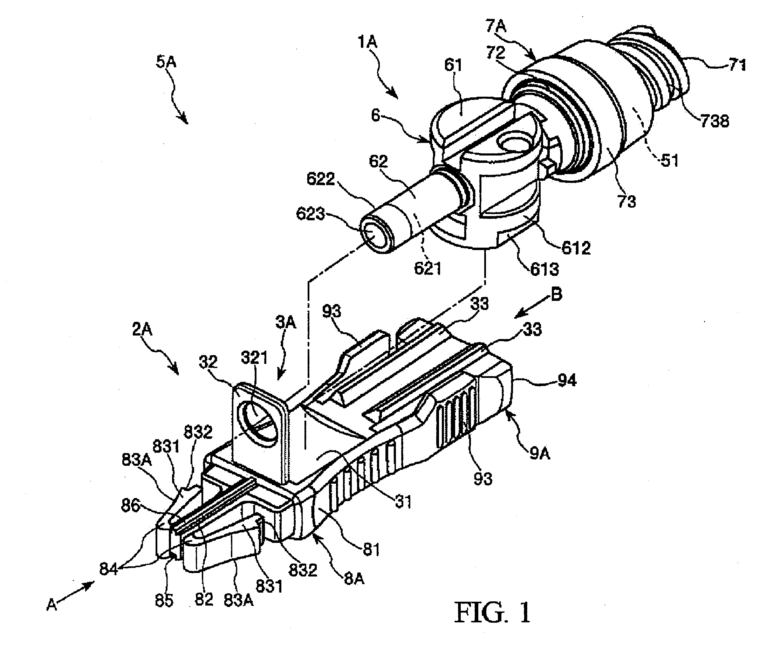

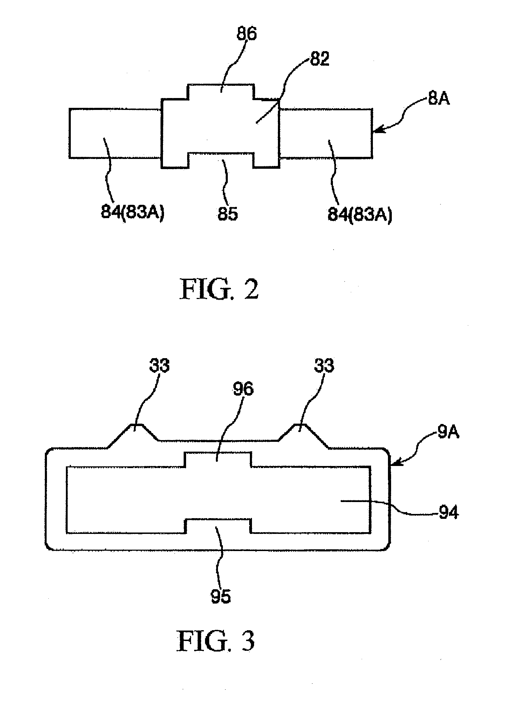

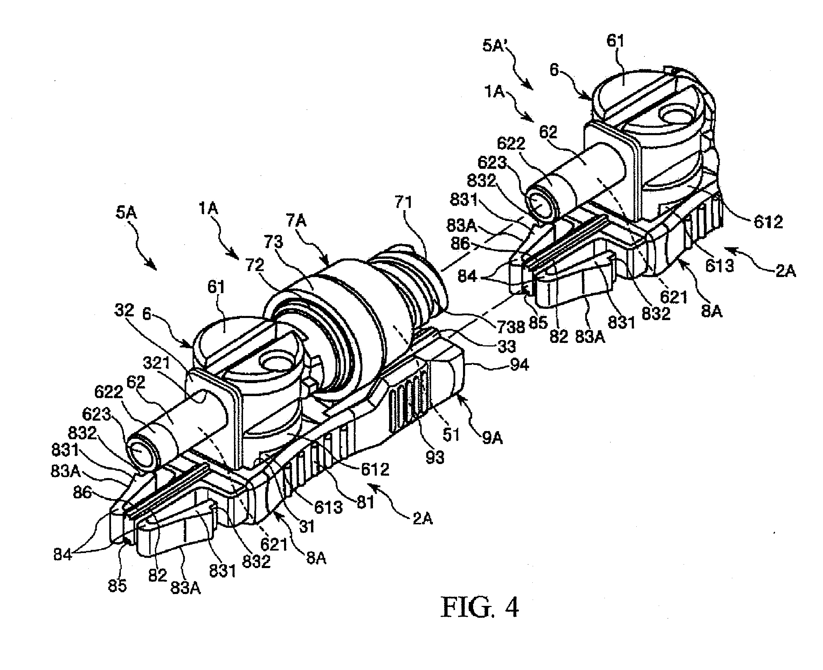

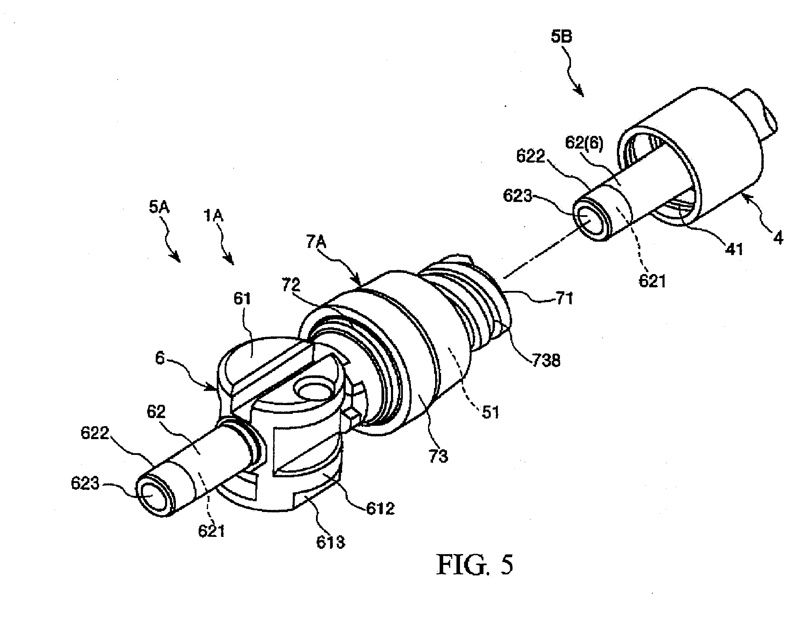

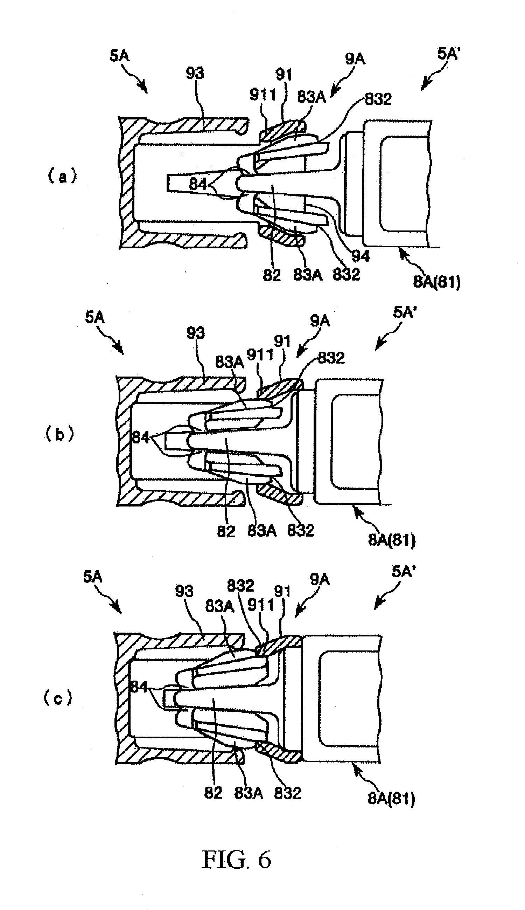

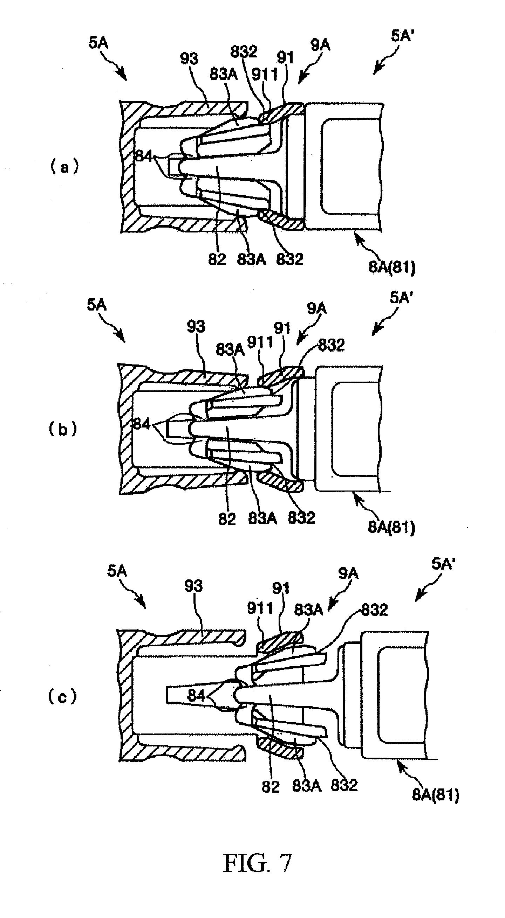

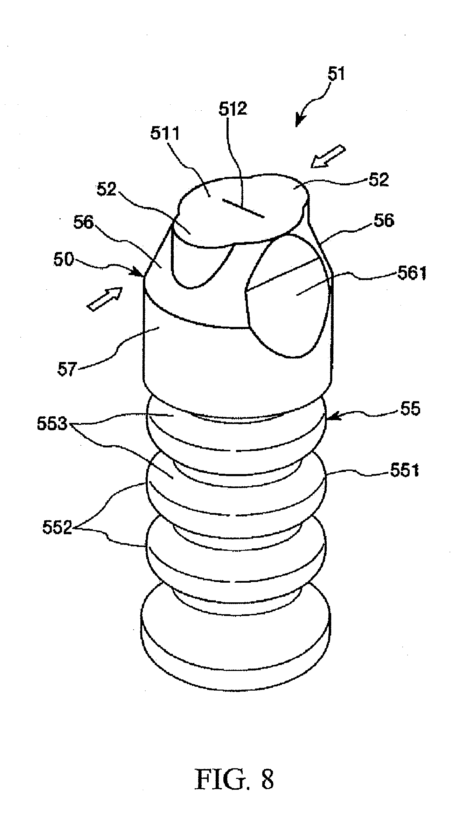

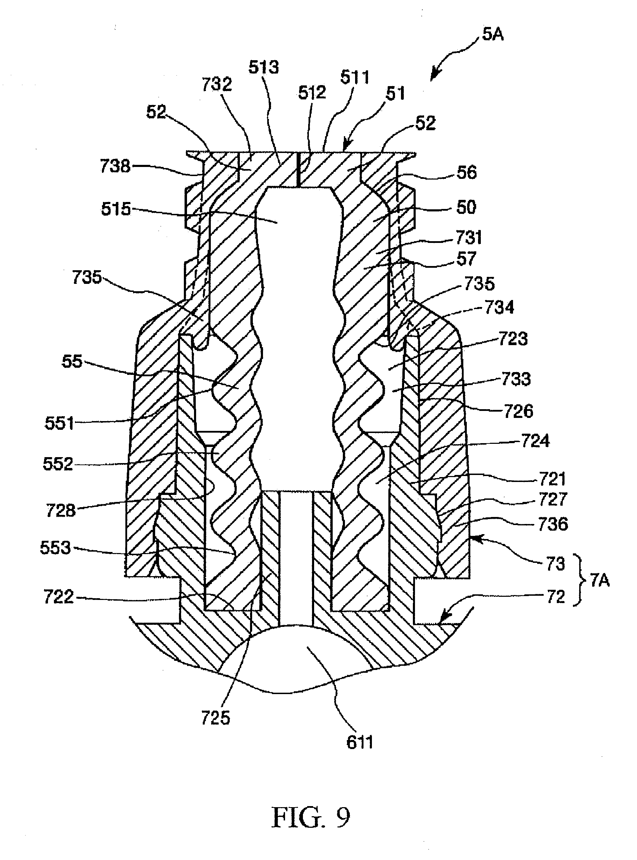

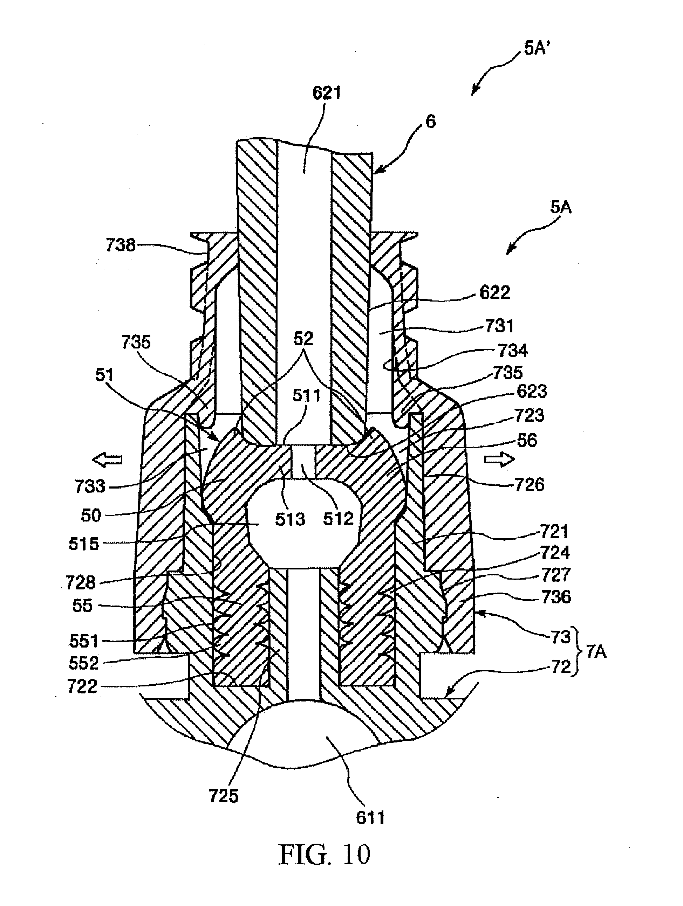

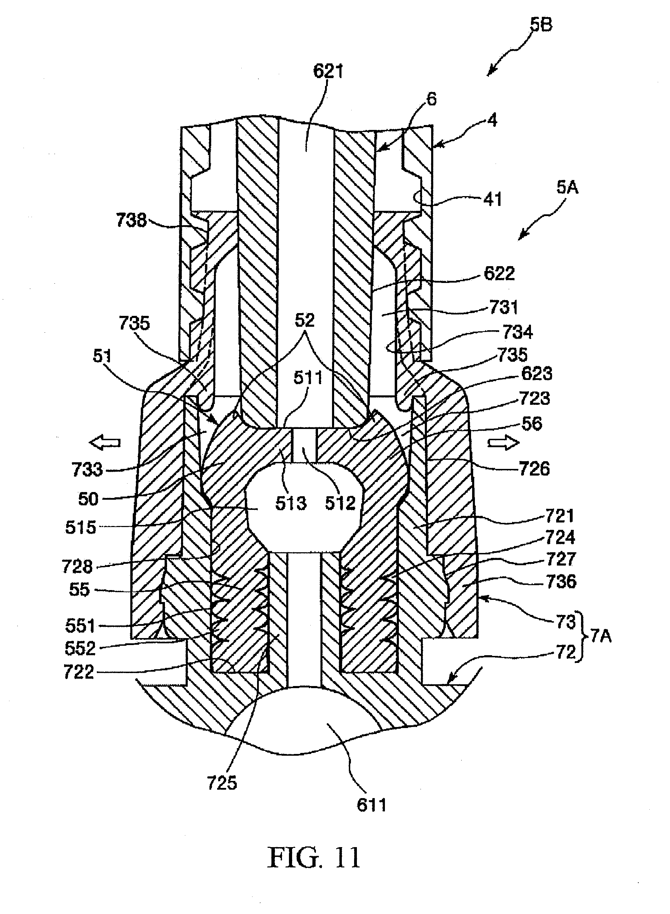

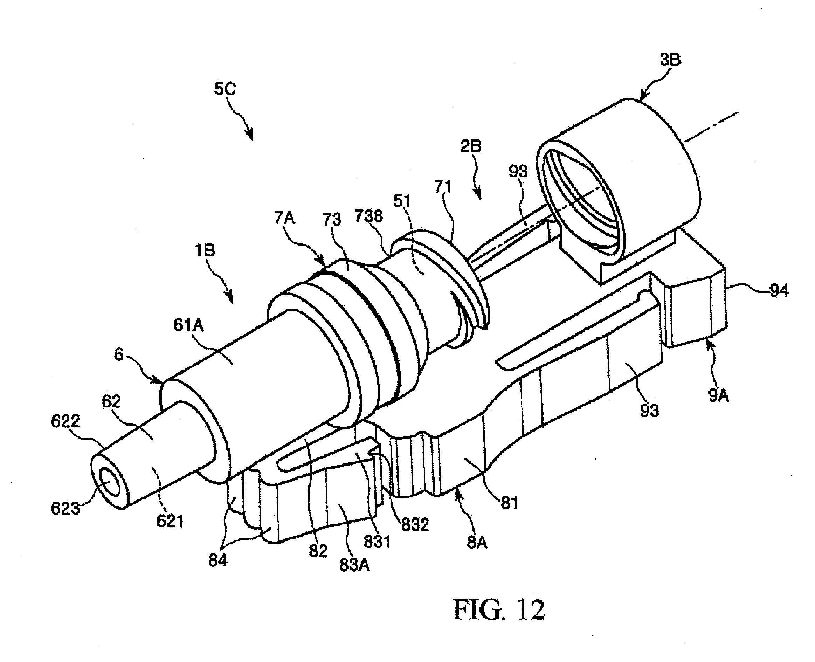

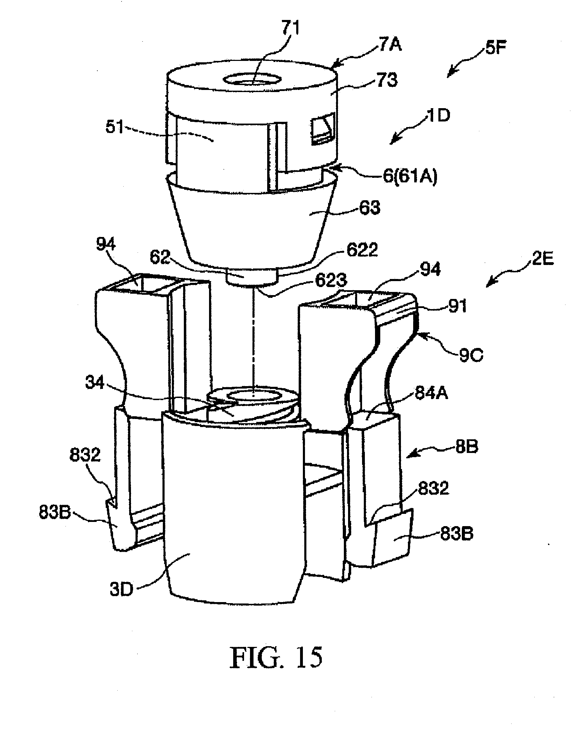

FIG. 1 is an exploded perspective view showing a first embodiment of the connector according to the present invention; FIG. 2 is a schematic view of the connector shown in FIG. 1, as viewed from the side of the arrow A in FIG. 1; FIG. 3 is a schematic view of the connector shown in FIG. 1, as viewed from the side of the arrow B in FIG. 1; FIGS. 4 and 5 are each perspective views showing an exemplary form in which the connector shown in FIG. 1 is used; FIG. 6 shows partial longitudinal sectional views, illustrating a process of connecting the connector (female lock section), when used in the form shown in FIG. 4; FIG. 7 shows partial longitudinal sectional views illustrating a process of canceling the connection of the connector (female lock section), when used in the form shown in FIG. 4; FIG. 8 is a perspective view of a valve element possessed by the connector shown in FIG. 1; FIG. 9 is a longitudinal sectional view of a female connector section of the connector shown in FIG. 1; FIG. 10 is a longitudinal sectional view showing a connected state of the connector (female connector section), when used in the form shown in FIG. 4; and FIG. 11 is a longitudinal sectional view showing a connected state of the connector (female connector section), when used in the form shown in FIG. 5. Incidentally, in the following description, for facilitating description, the right upper side in FIGS. 1 to 5 (also in FIGS. 12 to 14 and 16) will be referred to as a "proximal" side, and the left lower side as a "distal" side. Also, the right side in FIGS. 6 and 7 will be referred to as a "proximal" side, and the left side as a "distal" side. Further, the upper side in FIGS. 8 to 10 (also in FIG. 15) will be referred to as a "proximal" side, and the lower side as a "distal" side.

The connector 5A shown in FIG. 1 serves to interconnect passages (e.g., tubes) through which an infusion passes, for example, when injecting (administering) the infusion into a living organism (patient). Incidentally, the term infusion includes all liquids that can be administered to living organisms, for example, liquid medicines, correcting electrolytes, and physiological saline. The connector 5A has a first structure 1A, and a second structure 2A. Configurations of such components will now be described below.

The first structure 1A includes a male connector section 6, a female connector section 7A, and a valve element 51.

Initially, the male connector section 6 will be described.

As shown in FIGS. 1 to 5, the male connector section 6 includes a hollow male connector section main body (columnar part) 61, and a tubular part 62 that communicates with the male connector section main body 61.

The male connector section main body 61 makes up a portion having a substantially cylindrical outside shape.

The male connector section 6 is provided, on the lower side in FIG. 1 (also in FIGS. 4 and 5), with a pair of deficit portions 613, where an outer peripheral portion 612 thereof is partly removed. Such deficit portions 613 each are formed as flat surfaces and are disposed in parallel with each other.

The tubular part 62 projects in the distal direction from the outer peripheral portion 612 of the male connector section main body 61. In other words, the center axis of the tubular part 62 is orthogonal to the center axis of the male connector section main body 61. The tubular part 62 includes a liquid passage (male-side cavity) 621 therein through which liquid passes, and a luer tapered part 622.

The liquid passage 621 communicates with the interior of the male connector section main body 61.

The luer tapered part 622 is formed on the outer peripheral portion of the male connector section 6 (tubular part 62), which is disposed on the side of an opening part 623, in such a manner that the outside diameter thereof gradually decreases toward the opening part 623.

Next, the female connector section 7A will be described.

The female connector section 7A makes up a portion to (into) which another male connector section 6, which has the same configuration as the male connector section 6, can be connected (fitted). Herein, the term "another male connector section 6" implies, for example, a male connector section 6 possessed by a connector 5A', as shown in FIG. 4, and a male connector section 6 possessed by a connector 5B, as shown in FIG. 5.

As shown in FIGS. 9 to 11, the female connector section 7A includes a female connector section main body 72, and a cap section (cap) 73.

As shown in FIG. 9 (also in FIGS. 10 and 11), the female connector section main body 72 is formed, at a distal portion thereof, with a valve element disposing section 721 having a bottomed cylindrical shape. The valve element disposing section 721 is formed with a second cavity (female-side cavity) 723 therein on the proximal side, and with a third cavity (female-side cavity) 724 on the distal side, which communicates with the second cavity 723. The inside diameter of the second cavity 723 is larger than that of a first cavity (female-side cavity) 731 formed in the cap section 73, to be described later, and the inside diameter of the third cavity 724 (an inner peripheral surface 728) is smaller than that of the second cavity 723. The inside diameter of the third cavity 724, preferably, is slightly larger than the outside diameter of a barrel part 55 (an outer peripheral surface 551) of the valve element 51, to be described later.

In addition, at a central portion on a bottom surface 722 of the female connector section main body 72, an internal projection 725 is provided, which is composed of a tubular body. As shown in FIG. 10 (also in FIG. 11), when the male connector section 6 of the connector 5A' is fitted into the first cavity 731 (connection port 732) and the valve element 51 begins to be pressed, the inside portion of the valve element 51 is supported by the internal projection 725, whereby buckling of the valve element 51 (bending of the valve element 51 into a V-shape) can be prevented from occurring. Further, when liquid passes through the connector 5A, stagnation of such liquid can be prevented.

In addition, a lumen of the internal projection 725 communicates with a passage 611 (a hollow portion of the male connector section main body 61), which is formed inside the male connector section main body 61 and through which liquid can pass. This ensures that the second cavity 723 and the third cavity 724 communicate with the passage 611 through the internal projection 725.

Further, at the distal side of an outer peripheral surface 726 of the valve element disposing section 721, a stepped part 727 is provided, which is set larger in diameter than the portion on the proximal side thereof.

The cap section 73, as shown in FIG. 9, is provided therein with a space (female-side cavity) that contains the valve element 51, and is coupled to the proximal side (valve element disposing section 721) of the female connector section main body 72.

The cap section 73 also is provided therein with the first cavity 731, in which a head part 50 of the valve element 51, to be described later, can be inserted, and a fitting part 733, which communicates with the first cavity 731 and is set larger in diameter than the first cavity 731.

The first cavity 731 has a shape that corresponds to the outer shape of the head part 50 of the valve element 51. In addition, the connection port (connecting part) 732, which connects with the male connector section 6 of the connector 5A' or with the connector 5B, is formed at the proximal side of the first cavity 731, and the diameter thereof is set smaller than the diameter of the first cavity 731 on the distal side.

The first cavity 731 is provided at an inner peripheral surface 734 thereof with a plurality of ribs 735, which extend along the axial direction and project in a radial direction of the first cavity 731. When the male connector section 6 of the connector 5A' or the connector 5B is connected to the connector 5A (connection port 732), the valve element 51 is supported by the ribs 735, whereby buckling of the valve element 51 (and falling off of the valve element 51) can be prevented from occurring. In addition, the number of ribs 735 is not particularly limited; for example, the number is preferably two to ten, and more preferably, four to eight.

A stepped part 736, in which the stepped part 727 of the valve element disposing section 721 is to be fitted, is formed on the distal side of the fitting part 733, and the diameter thereof is set to be larger than the diameter of the fitting part 733 on the proximal side. In addition, the inside diameter of the stepped part 736, preferably, is approximately equal to or slightly smaller than the outside diameter of the stepped part 727 of the valve element disposing section 721. This enables firm fitting (coupling) (liquid-tight contact) between the cap section 73 (stepped part 736) and the female connector section main body 72 (stepped part 727), and thus liquid inside the connector 5A can be prevented from leaking. Further, when the cap section 73 and the female connector section main body 72 are coupled together, the first cavity 731 and the second cavity 723 communicate with each other, and the valve element 51 can be disposed (contained) within the space composed of the first cavity 731, the second cavity 723, and the third cavity 724.

In addition, the cap section 73 (female connector section 7A) is formed with a male screw part 738 at an outer peripheral portion thereof.

Incidentally, the method for fixing the female connector section main body 72 and the cap section 73 to each other is not limited to the aforementioned fitting. For example, the method may be performed by caulking, adhesion with an adhesive, fusing such as heat fusing and ultrasonic fusing, or the like.

As shown in FIG. 1 (also in FIGS. 4 and 5), the female connector section 7A is disposed on the proximal side in relation to the male connector section main body 61 (male connector section 6). In other words, the female connector section 7A is disposed such that the center line thereof is parallel to the center line of the male connector section 6, and so that an opening part 71 of the female connector section 7A and an opening part 623 of the male connector section 6 are oriented in opposite directions. This ensures that, for example, when the connectors 5A and 5A' are connected to each other, the connectors 5A and 5A' can be disposed substantially rectilinearly.

As shown in FIGS. 9 to 11, the valve element 51 is contained (fixed) in the female connector section 7A.

The valve elements 51 are each formed from an elastic material. Examples of suitable elastic materials include various rubber materials such as natural rubber, isoprene rubber, butadiene rubber, styrene-butadiene rubber, nitrile rubber, chloroprene rubber, butyl rubber, acrylic rubber, ethylene-propylene rubber, hydrin rubber, urethane rubber, silicone rubber, fluororubber, etc., and various thermoplastic elastomers based on styrene, polyolefin, polyvinyl chloride, polyurethane, polyester, polyamide, polybutadiene, trans-polyisoprene, fluororubber, chlorinated polyethylene, or the like, which may be used either singly or in a mixture of two or more of them. When such an elastic material is used, an appropriate elasticity can be imparted to a top face 511 of the valve element 51, and therefore, the top face 511 can be placed in secure contact with the opening part (end part) 623 of the male connector section 6 of the connector 5A' or the connector 5B (see FIGS. 10 and 11).

As shown in FIG. 9, the valve element 51 includes the head part 50, and the barrel part 55, which is provided (formed) on the distal side of the head part 50.

The head part 50 has a bottomed cylindrical shape, and is formed with a cavity 515 therein through which liquid can pass, together with a slit 512 that extends from the flat top face 511 (bottom part 513) to reach the cavity 515. The slit 512 is formed substantially in the shape of a straight line segment. The simple shape of the slit 512 enables easier (more assured) opening of the slit 512. In addition, the flat shape of the top face 511 permits the top face 511 (slit 512) to be disinfected easily.

As shown in FIG. 9, the top face 511 is exposed from the opening part 71 of the female connector section 7A. Further, the top face 511 is located substantially flush with an end face of the female connector section 7A.

As shown in FIG. 8, the head part 50 includes a tapered part 56 having an outside diameter in the vicinity of the top face 511 that gradually increases (in the axial direction) toward the barrel part 55, and a constant outside diameter part 57 provided at the distal end of the tapered part 56.

In addition, the tapered part 56 is formed with cutout portions 561 therein where the tapered part 56 is partly removed. Specifically, the head part 50 is formed with such cutout portions 561, where material is removed in areas ranging from the tapered part 56 to the constant outside diameter part 57.

This ensures that when the male connector section 6 connected to (inserted in) the connector 5A is pulled off, the valve element 51 (head part 50) can enter easily into the first cavity 731 of the cap section 73, and therefore, the slit 512 can be closed more reliably.

In addition, the head part 50 is provided with two protuberant contact portions 52, which are pressed at times when the slit 512 is closed. The two contact portions 52 are respectively formed in the vicinity of the top face 511 of the head part 50, and project in directions opposite to the directions (directions of the arrows in FIG. 8) in which the slit 512 is closed.

The presence of the contact portions 52 ensures that when the head part 50 is inserted into the first cavity 731 of the cap section 73, the inner peripheral surface 734 of the first cavity 731 presses against the contact portions 52, so that the slit 512 can be closed more reliably (see FIG. 9). In addition, this makes it possible to enhance pressure resistance against the pressure of the liquid inside the connector 5A (internal pressure).

When the male connector section 6 of the connector 5A' or the connector 5B is not connected, the head part 50, configured as described above, is inserted into the first cavity 731 of the cap section 73, with the slit 512 being closed (see FIG. 9).

As shown in FIG. 8, the barrel part 55 is composed of a bellows-like cylindrical body. Specifically, the barrel part 55 has a bellows-like outside shape, in which large diameter ring portions 552 and small diameter ring portions 553 are alternately arrayed along the axial direction. Such a barrel part 55 functions as a deforming section (urging means), for urging the valve element 51 from the distal side toward the proximal side (i.e., in the direction in which the head part 50 is inserted into the first cavity 731 in the cap section 73).

Since the barrel part 55 functions as a deforming section, it is unnecessary to separately provide a component part on the connector 5A serving as an urging means. Therefore, a reduction in the number of component parts, and simplification in structure, can be realized.

In addition, although the barrel part 55 provides most of the restoring force for restoring the valve element 51 from the distal side toward the proximal side, the head part 50 may also provide a portion of the restoring force.

The material constituting the first structure 1A (exclusive of the valve element 51) is not particularly limited. Examples of suitable materials that can be used include various resins, such as polyvinyl chloride, polyethylene, polypropylene, cyclic polyolefins, polystyrene, poly-(4-methylpentene-1), polycarbonate, acrylic resin, acrylonitrile-butadiene-styrene copolymer, polyesters including polyethylene terephthalate, polyethylene naphthalate, etc., butadiene-styrene copolymer, and polyamides (e.g., 6-nylon, 6,6-nylon, 6,10-nylon, 12-nylon).

As shown in FIG. 1, the second structure 2A includes a male lock section 8A, a female lock section 9A, and an installation section 3A. The second structure 2A can be installed on (mounted onto) the first structure 1A. Hereinafter, the condition in which the second structure 2A is installed on the first structure 1A will be referred to as "the installed condition."

First, the male lock section 8A will be described.

The male lock section 8A includes a base part (male lock section main body) 81, a projected part 82 that projects from the base part 81, a pair of claw parts 83A provided on the projected part 82, and urging parts 84 for urging the claw parts 83A.

The base part 81 has an elongate shape. On the distal side of the base part 81, the projected part 82 is formed and projects in the distal direction.

On both lateral sides of the projected part 82, the claw parts 83A are disposed, respectively.

In the installed condition, the claw parts 83A project in a direction opposite to the male connector section 6 (tubular part 62), i.e., project in the proximal direction. As a result, the male lock section 8A as a whole has a shape in which the width thereof gradually decreases in the distal direction (see, for example, FIGS. 6 and 7). Therefore, when the male lock section 8A of the connector 5A is inserted into a female lock section 9A of a connector other than the connector 5A, the inserting operation can be carried out easily.

In addition, respective end parts 831 of both of the claw parts 83A can move toward and away from each other.

The claw parts 83A are provided, on the end parts 831 thereof, with male-side engaging parts 832, which are formed as if the end parts 831 thereof were partially cut out (lost).

The urging parts 84 are disposed respectively between the projected part 82 and the claw part 83A. The urging parts 84 urge the claw parts 83A so as to move the respective end parts 831 (male-side engaging parts 832) of both of the claw parts 83A away from each other.

In the installed condition, the male lock section 8A thus configured is located at a position corresponding to (adjacent to) the male connector section 6 (see FIG. 4). In addition, the male lock section 8A and the male connector section 6 are oriented respectively in the same direction. In other words, the directions (projecting directions) in which the sections are connected are parallel to each other.

Next, the female lock section 9A will be described below.

As shown in FIG. 4, the female lock section 9A forms a portion to which another male lock section 8A, having the same configuration as the male lock section 8A, can be coupled. Herein, the term "another male lock section 8A" implies a male lock section 8A, for example, which is possessed by the connector 5A' (see FIG. 4).

The female lock section 9A is provided on the proximal side relative to the base part 81 (male lock section 8A). The female lock section 9A is substantially tubular in overall shape.

As shown in FIGS. 4, 6 and 7, the female lock section 9A includes a female-side engaging part 91, which is capable of engagement with each of the male-side engaging parts 832 of the male lock section 8A of the connector 5A', and operating parts 93 that are capable of operating the claw parts 83A of the male lock section 8A of the connector 5A'.

As shown in FIG. 6 (also in FIG. 7), the female-side engaging part 91 is formed such that an inner peripheral portion of the female lock section 9A on the opening part 94 side thereof gradually diminishes along the distal direction. As shown in FIG. 6(c), through engagement of the end part 911 of the female-side engaging part 91 with the male-side engaging parts 832 of the male lock section 8A of the connector 5A', the connector 5A (female lock section 9A) and the connector 5A' (male lock section 8A) are placed in a locked condition.

The operating parts 93 are composed of small pieces provided on the distal side in relation to the female-side engaging part 91. The operating parts 93 operate (press) both claw parts 83A of the connector 5A' (male lock section 8A) in the locked condition, so that the end parts 831 of the claw parts 83A come closer to each other.

Besides, the operating parts 93 are formed such that outer surfaces thereof are substantially flush with the outer surface of the female-side engaging part 91 (see FIG. 6). This makes it possible to prevent the operator from touching (pressing) the operating parts 93 unintentionally. In other words, unintentional release of the locked condition can be prevented from occurring.

In the installed condition, the female lock section 9A thus configured is located corresponding to (adjacent to) the female connector section 7A (see FIG. 4). In addition, the female lock section 9A and the female connector section 7A are oriented in the same direction. In other words, the directions of connection (formation directions) of the sections are parallel to each other.

Next, the installation section 3A will be described below.

The installation section 3A forms a portion on which the first structure 1A can be installed (see FIGS. 1 and 4). The installation section 3A, as shown in FIG. 1, is composed of a recess 31 formed on an upper surface of the base part 81 of the male lock section 8A, and a small plate-like piece 32 that projects from the upper surface.

The recess 31 is a portion, which is arcuate in cross-sectional shape. In the installed condition, the male connector section main body 61 of the first structure 2A is fitted into the recess 31 (see FIG. 4).

In addition, the small plate-like piece 32 is disposed in an erect manner from the base part 81 of the male lock section 8A. The small plate-like piece 32 has a through-hole 321 therein, which penetrates through the small plate-like piece 32. The inside diameter of the through-hole 321 is set approximately equal to the outside diameter of the tubular part 62, proximate to a base portion that lies contiguous to the male connector section main body 61. As shown in FIG. 4, in the installed condition, the tubular part 62 of the male connector section 6 is fitted (inserted) into the through-hole 321.

Thus, at the installation section 3A, in the installed condition, the first structure 1A is fixed at two locations, namely, at the male connector section main body 61 and at the tubular part 62 of the male connector section 6. This ensures that, once the installed condition has been attained in the connector 5A, the first structure 1A and the second structure 2A cannot be separated, or stated otherwise, the second structure 2A cannot be detached from the first structure 1A. Therefore, while the connector 5A is being used, i.e., while the connector 5A and the connector 5A' are connected to each other, unintentional disconnection of the second structure 2A from the first structure 1A of the connector 5A can reliably be prevented from occurring.

In addition, on the proximal side of the recess 31, two guide rails 33, 33 are formed along the longitudinal direction of the second structure 2A. When the connector 5A is placed in the installed condition, the deficit portions 613 of the male connector section main body 61 of the first structure 1A can be set respectively along the guide rails 33. Further, when the deficit portions 613 reach the terminal ends of the guide rails 33, the male connector section main body 61 is disposed in the recess 31. Substantially simultaneously therewith, the tubular part 62 is fitted into the hole 321 of the small plate-like piece 32. Thus, the guide rails 33 serve a function to assist the operation of obtaining the installed condition. This ensures that the operation for attaining the installed condition can be carried out smoothly and easily.

Further, the recess 31 in the second structure 2A has a curved surface, which is substantially the same as a bottom surface of the male connector section main body 61 of the first structure 1A. This ensures that, even after the installed condition has been attained, the second structure 2A can be rotated around the first structure 1A, with the tubular part 62 of the first structure 1A serving as an axis of rotation.

In addition, the material constituting the second structure 2A is not particularly limited. For example, constituent materials, such as those mentioned above in relation to the first structure 1A, can be used.

Next, a method of using the connector 5A will be described below. Prior to describing the method of use, the mating body, which is to be connected to the connector 5A, shall be described. Examples of the mating body include the aforementioned connector 5A' shown in FIG. 4, as well as the connector 5B shown in FIG. 5.

As shown in FIG. 4, the connector 5A' includes a male connector section 6 and a male lock section 8A, which are the same as those of the connector 5A. Further, on the connector 5A', the positional relationship between the male connector section 6 and the male lock section 8A is the same as that of the connector 5A. More specifically, the male connector section 6 and the male lock section 8A project in the same direction and lie adjacent to each other.

As shown in FIG. 5, the connector 5B does not have a male lock section 8A the same as that of the connector 5A, but rather has a male connector section 6, which is the same as that of the connector 5A, and further has a luer lock member 4 arranged on an outer peripheral side of the tubular part 62 of the male connector section 6. The luer lock member 4 has a bottomed cylindrical shape. A bottom part of the luer lock member 4 is rotatably supported, so as to be rotatable relative to the tubular part 62 of the male connector section 6. In addition, the luer lock member 4 is provided, on an inner peripheral surface thereof, with a female screw part 41, which is capable of screw engagement with the male screw part 738 of the female connector section 7A of the connector 5A.

Meanwhile, in the connector 5A, the first structure 1A can be used selectively, according to the configuration of the mating body, either with the second structure 2A installed thereon (in the installed condition) (the condition shown in FIG. 4), without the second structure 2A installed thereon (the condition shown in FIG. 5), or used in a combination of one with the second structure 2A installed thereon and another without the second structured 2A installed thereon (not shown).

In other words, when using the connector 5A, in the event that the mating body for the connector 5A comprises the connector 5A' (this case will hereinafter be referred to as a "first case"), the first structure 1A can be used with the second structure 2A installed thereon (in the installed condition). In addition, in the event that the mating body for the connector 5A comprises the connector 5B (this case will hereinafter referred to as a "second case"), the first structure 1A can be used without the second structure 2A installed thereon (in the non-installed condition). Further, in the event that the mating body for the connector 5A, which already is connected with another connector or tube, comprises the connector 5B (this case will hereinafter be referred to as a "third case"), the first structure 1A can be used with the second structure 2A installed thereon (in the installed condition).

Next, a process of connecting (form of use (method of use)) the respective connectors in each of the aforementioned cases will be described below.

### [1] First Case

As shown in FIG. 4, the connector 5A is in the installed condition. Starting from a condition in which the connector 5A and the connector 5A' both in the installed condition are separated from each other, as shown in FIG. 6(a), the male lock section 8A of the connector 5A' is brought closer in proximity to the female lock section 9A of the connector 5A.

While the male lock section 8A of the connector 5A' is inserted into the female lock section 9A of the connector 5A, as shown in FIG. 6(b), the female-side engaging part 91 of the connector 5A presses against the claw parts 83A of the connector 5A' in opposition to the urging forces of the urging parts 84 of the connector 5A'.

Starting from this condition, the male lock section 8A of the connector 5A' and the female lock section 9A of the connector 5A are further brought toward each other, whereupon the male-side engaging parts 832 of the connector 5A' engage with the female-side engaging part 91 of the connector 5A, i.e., the connector 5A (female lock section 9A) and the connector 5A' (male lock section 8A) are placed in a locked condition (see FIG. 6(c)).

The respective second structures 2A of the connector 5A and the connector 5A' can be rotated respectively around the first structures 1A. This ensures that, even in the case where, for example, the position (disposition) of the connector 5A or the connector 5A' is to be changed, such that the position of the second structure 2A relative to the first structure 1A also has to be changed (for example, where the second structure 2A initially located at the bottom surface of the first structure 1A is to be repositioned and located at the upper surface thereof), the respective second structures 2A can be connected in aligned positions, without twisting the tube(s) that are connected therewith.

In addition, when the connector 5A and the connector 5A' are placed in the locked condition, the second structure 2A of the connector 5A and the second structure 2A of the connector 5A' can be rotated, while remaining in the locked condition, respectively around the first structure 1A of the connector 5A and the first structure 1A of the connector 5A'. This ensures that, for example, even when the position (disposition) the connector 5A and the connector 5A' in the connected condition is to be changed, such that the position of the second structure 2A relative to the first structure 1A also has to be changed (for example, where the second structure 2A initially located at the bottom surface of the first structure 1A is to be repositioned and located at the upper surface thereof), such a change in position can be achieved without twisting the tube(s) that are connected thereto.

Further, as shown in FIG. 10, in the locked condition, the opening part 623 of the male connector section 6 of the connector 5A' presses against the top face 511 of the head part 50 in the axial direction of the connector 5A. As a result, the barrel part 55 is deformed (compressed) in the axial direction, and the head part 50 moves from the first cavity 731 into the second cavity 723. The head part 50, which formerly resided in the first cavity 731 restricted by the inner peripheral surface 734 of the first cavity 731, now moves into the second cavity 723, whereby the restriction imposed on the outer peripheral surface of the head part 50 is released. Consequently, as a result of being compressed in the axial direction, the head part 50 can be enlarged (i.e., sufficiently deformed) in diameter in the directions of the arrows in FIG. 10. Therefore, the slit 512 can be opened sufficiently and reliably. In addition, as a result, the liquid passage 621 in the male connector section 6 of the connector 5A' and the lumen (hollow portion) of the internal projection 725 of the female connector section 7B of the connector 5A communicate mutually through the valve element 51 (slit 512). In other words, such elements are connected to permit flow of liquid, so that liquid can pass smoothly therethrough, for example, during an infusion, transfusion, nutrient dosing, or the like.

In addition, in the locked condition, the opening part 623 of the male connector section 6 of the connector 5A' presses against the head part 50 of the valve element 51 in opposition to the urging force of the barrel part 55 of the valve element 51, so that the opening part 623 of the male connector section 6 and the top face 511 of the head part 50 (valve element 51) make firm contact with each other (see FIG. 10). Owing thereto, liquid tightness of the connection between the male connector section 6 and the female connector section 7B can be maintained, i.e., the sections can be connected assuredly in a liquid-tight manner.

Further, the locked condition between the connector 5A' and the connector 5A is released by operating the operating parts 93 of the connector 5A.

In other words, starting from the locked condition shown in FIG. 7(a), the claw parts 83A of the connector 5A' are pressed by the operating parts 93 of the connector 5A, as shown in FIG. 7(b). As a result, engagement between the female-side engaging part 91 of the connector 5A and the male-side engaging parts 832 of the connector 5A' is released (canceled).

Thereafter, as shown in FIG. 7(c), the connector 5A' is pulled, whereby the connector 5A' and the connector 5A are separated away from each other.

### [2] Second Case

Starting from a condition in which the first structure 1A (connector 5A) and the connector 5B are separated from each other, the male connector section 6 (luer lock member 4) of the connector 5B is moved closer in proximity toward the female connector section 7A of the first structure 1A. Attendant therewith, the male screw part 738 of the female connector section 7A of the first structure 1A and the female screw part 41 of the luer lock member 4 of the connector 5B are screw-engaged with each other (see FIG. 11). As a result, the first structure 1A and the connector 5B are placed in the locked condition.

In addition, in the locked condition, the first structure 1A and the connector 5B are connected to each other in a liquid-tight manner, in the same way as in the first case above (see FIG. 11). As a result, liquid can pass through the first structure 1A and the connector 5B, for example, during an infusion, transfusion, nutrient dosing, or the like.

Further, releasing of the locked condition between the first structure 1A and the connector 5B is carried out by loosening the luer lock member 4 of the connector 5B (by releasing screw-engagement thereof with the female connector section 7A of the first structure 1A).

### [3] Third Case

Initially, a connector 5A is in the installed condition, and is connected to a tube or the like, so that a state in which liquid or the like can flow therethrough has already been attained. From this point, in the same manner as in the second case, starting from the condition in which the connector 5A and a connector 5B are separated from each other, the connector 5A and the connector 5B can be placed in the locked condition.

As has been mentioned above, when using the connector 5A, the connector 5A is modified as required, according to the mating body, which is to be connected to the connector 5A. Specifically, the manner of using the connector 5A can be selected between usage thereof in an installed condition and usage thereof in a non-installed condition. This ensures that the connector 5A and the mating body can be connected to each other assuredly, so that liquid can flow through them in both directions.

Thus, in usage thereof, the connector 5A is rich in versatility.

Incidentally, as shown in FIG. 2 (also in FIG. 1), the projected part 82 of the male lock section 8A, preferably, is formed with a groove 85 and a rib 86, extending along the longitudinal direction thereof. In addition, as shown in FIG. 3, the female lock section 9A, preferably, is formed at the opening part 94 thereof with a rib 95 and a groove 96, which extend along the longitudinal direction of the female lock section 9A.

The groove 85 is formed as a recess in a surface on the lower side of the projected part 82, as shown in FIG. 2. The rib 86 is formed so as to project from a surface on the upper side of the projected part 82 in FIG. 2.

The rib 95 is formed so as to project from a surface on the lower side of the opening part 94, as shown in FIG. 3. The groove 96 is formed as a recess in a surface on the upper side of the opening part 94 in FIG. 3.

In the first case, when the connector 5A and the connector 5A' are connected, the rib 86 of the male lock section 8A of the connector 5A' enters (is fitted) into the groove 96 in the female lock section 9A of the connector 5A. Concurrently therewith, the rib 95 of the female lock section 9A of the connector 5A enters (is fitted) into the groove 85 in the male lock section 8A of the connector 5A'.

Since such a connection can be achieved, it is possible to prevent an operator from making a mistake in grasping the orientation, in the vertical direction as shown in FIG. 4, of the male lock section 8A of the connector 5A' relative to the female lock section 9A of the connector 5A.

### <Second Embodiment>

FIG. 12 is an exploded perspective view showing a second embodiment of the connector according to the present invention.

The second embodiment of the connector according to the present invention will be described below with reference to FIG. 12. The following description shall focus on differences from the above-described embodiment, and descriptions of the same items, which have already been described above, will be omitted.

The present embodiment is the same as the first embodiment, except for certain differences in the configuration of the installation section.

A first structure 1B of a connector 5C, as shown in FIG. 12, has a configuration in which a male connector section main body 61A is set to be larger in diameter than a tubular part 62 thereof.

In addition, a second structure 2B includes an installation section (ring-shaped section) 3B, onto which the first structure 1B is to be installed. The installation section 3B is arranged in the vicinity of a female lock section 9A. Further, the installation section 3B is ring-like in shape, and the inside thereof is formed in the shape of a screw, which is capable of engagement with a female luer lock. The center axis thereof is parallel to the longitudinal direction of the second structure 2B.

In the installed condition of the connector 5C, an outer peripheral portion of a female connector section 7A of the first structure 1B is fitted into the installation section 3B of the second structure 2B. As a result, the second structure 2B becomes affixed reliably to the first structure 1B. Therefore, once the installed condition has been established, the first structure 1B and the second structure 2B cannot be separated from each other. This ensures that, while the connector 5B is being used in the installed condition, unintentional disengagement of the second structure 2B from the first structure 1B can be prevented from occurring.

Naturally, the connector 5C can also be used in a non-installed condition.

### <Third Embodiment>

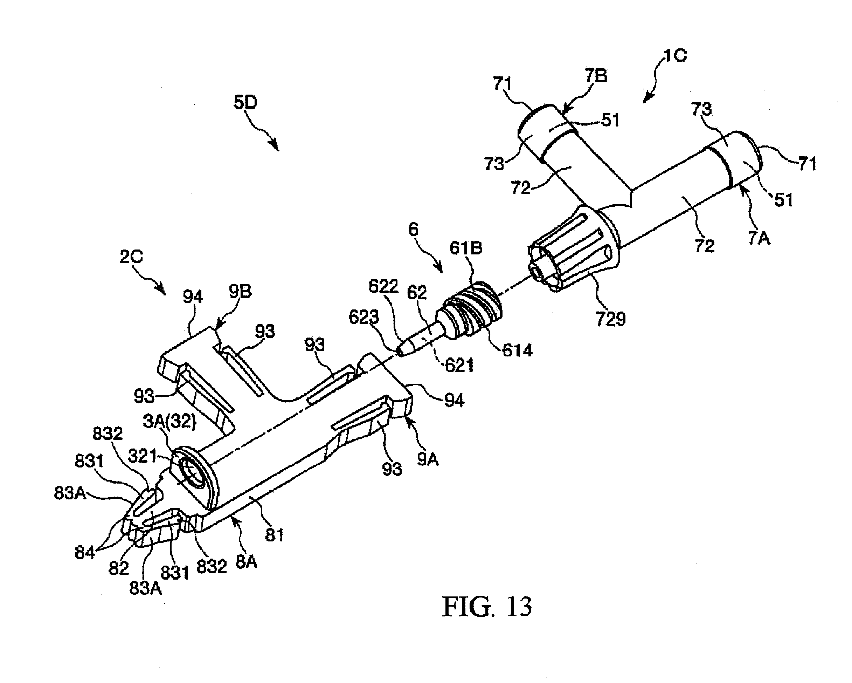

FIG. 13 is an exploded perspective view showing a third embodiment of the connector according to the present invention.

The third embodiment of the connector according to the present invention will be described below with reference to FIG. 13. The following descriptions will focus on differences from the above-described embodiments, and descriptions of the same items, which have already been described above, shall be omitted.

The present embodiment is the same as the above-described first embodiment, except for certain differences in the numbers of the respective female connector section(s) and female lock section(s).

A first structure 1C of a connector 5D further has a female connector section 7B, as shown in FIG. 13, in addition to a female connector section 7A. The female connector section 7B is the same as the female connector section 7A in configuration, and therefore, descriptions thereof have been omitted.

The female connector section 7B is provided in a direction, which is different from that of the female connector section 7A. Specifically, the female connector section 7B is disposed such that a center line thereof is substantially orthogonal to the center line of the female connector section 7A (also that of the male connector section 6).

The presence of the female connector sections 7A and 7B having such configurations ensures that liquid(s) can be fed into the male connector section 6 from two different directions, and also, that liquid can branch from the male connector section 6 in two different directions.

In addition, the first structure 1C has a configuration in which the male connector section 6 can be attached to and detached from the female connector section 7A.

More specifically, in the first structure 1C, a tubular male connector section main body 61B of the male connector section 6 is formed with a male screw part 614 on an outer peripheral portion thereof, and a female connector section main body 72 of the female connector section 7A is formed with a female screw part 729 on the outer peripheral side thereof. By screw engagement between the male screw part 614 and the female screw part 729, as well as disengagement from each other, the male connector section 6 can be attached to and detached from the female connector section 7A.

Further, a second structure 2C also includes a female lock section 9B, in addition to a female lock section 9A. The female lock section 9B is the same in configuration as the female lock section 9A, and therefore, detailed descriptions thereof have been omitted.

In the installed condition, the female lock section 9B is arranged at a position corresponding to the female connector section 7B of the first structure 1C. Specifically, the female lock section 9B is provided so that a center line thereof is substantially orthogonal to the center line of the female lock section 9A (also that of a male lock section 8A).

Incidentally, the number of female connector sections and female lock sections is not limited to two. For example, the number of each may be three or more.

### <Fourth Embodiment>

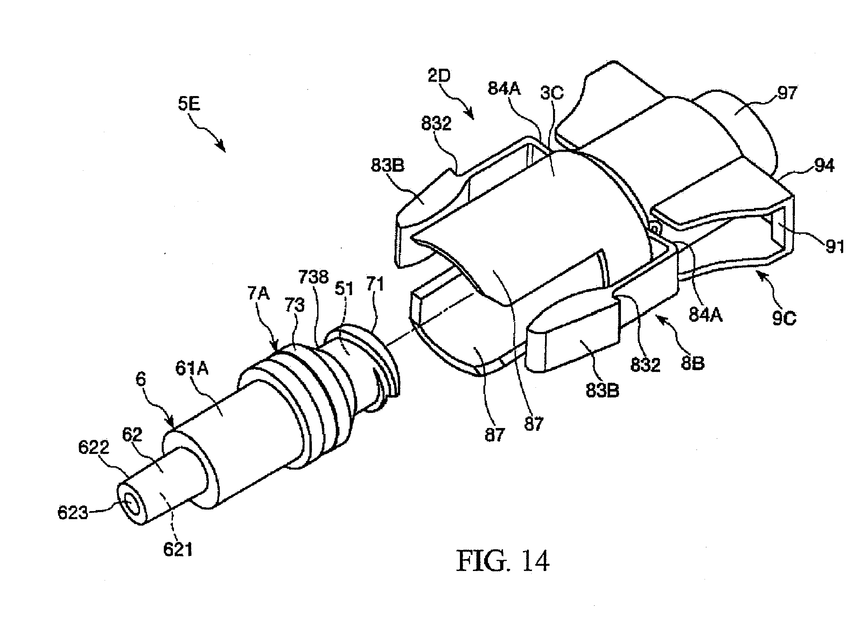

FIG. 14 is an exploded perspective view showing a fourth embodiment of the connector according to the present invention.

Next, referring to this figure, the fourth embodiment of the connector according to the present invention will be described below. The following description will focus on differences from the above-described embodiments, and descriptions of items, which are the same as those described above, will be omitted.

The fourth embodiment is the same as the second embodiment described above, except for differences in the shape of a second structure.

A second structure 2D of a connector 5E, as shown in FIG. 14, has a cylindrical installation section 3C, and a male lock section 8B and a female lock section 9C, which are disposed on the outer peripheral side of the installation section 3C.

In an installed condition, an outer peripheral portion of a female connector section 7A of a first structure 1B is fitted into the installation section 3C.

The male lock section 8B has a pair of claw parts 83B, 83B, arranged oppositely to each other with the installation section 3C intervening therebetween, and urging parts 84A for urging the claw parts 83B, respectively.

The claw parts 83B are each composed of a small plate-like piece, which projects in the distal direction, i.e., projects in the same direction as the male connector section 6 in the installed condition.

In addition, each of the claw parts 83B is formed with a stepped part, where the thickness thereof abruptly changes at an intermediate portion. The stepped part is a portion, which functions as a male-side engaging part 832 for engagement with another female lock section 9C, having the same configuration as that of the female lock section 9C.

The urging part 84A is located between the installation section 3C and each of the claw parts 83B, coupling the claw parts 83B to each other.

In the male lock section 8B thus configured, in certain situations, the claw parts 83B are moved toward each other in opposition to urging forces of the urging parts 84A, whereas in other situations, the claw parts 83B, which have been brought closer to each other, are moved away from each other by the urging parts 84A.

In addition, the male lock section 8B ensures that the connector 5E, in the installed condition, is securely placed in the locked condition in relation to a mating body, which has another female lock section 9C.

Further, the male lock section 8B includes a pair of flange parts 87, which are curved, and small and plate-like in shape, and which cover an outer side of a tubular part 62 in the installed condition. Since the outer side of the tubular part 62 is covered by the flange parts 87 in the installed condition, it is possible to prevent the tubular part 62 from being directly touched by a fingertip or the like, thereby protecting the tubular part 62 when carrying out a connecting operation or the like.

In addition, the female lock section 9C has a tubular flange-receiving part 97, over which flange parts 87 of another connector (not shown), which is the same as the connector 5E in the installed condition, are to be fitted. When the connector 5E in the installed condition and another connector the same as the connector 5E are connected to each other, the flange parts 87 of the other connector are fitted over the flange-receiving part 97 of the connector 5E, whereby the connector 5E is forcibly positioned at a given orientation. When the insertion-like fitting is advanced further, the tubular part 62 of the other connector is inserted into an opening part 71 of the connector 5E so as to be positioned therein. This ensures that upon connection thereof, positioning can be performed easily.

The female lock section 9C includes a portion for connection with another male lock section 8B, which has the same configuration as that of the male lock section 8B. The female lock section 9C includes female-side engaging parts 91 for engagement with male-side engaging parts 832 of the other male lock section 8B.

Due to the female lock section 9C, in the installed condition, the connector 5E is securely placed in the locked condition in relation to a mating body having the other male lock section 8B.

### <Fifth Embodiment>

FIG. 15 is an exploded perspective view showing a fifth embodiment of the connector according to the present invention.

Referring to the figure, the fifth embodiment of the connector according to the present invention will be described below. The following description will focus on differences from the above-described embodiments, and descriptions of items, which are the same as those described above, will be omitted.

The fifth embodiment is the same as the fourth embodiment above, except for a difference in the configuration of an installation section.

A first structure 1D of a connector 5F, as shown in FIG. 15, is provided with a female screw part 63 on an outer peripheral portion of a male connector section 6.

In addition, an installation section 3D of a second structure 2E is provided, at the inner peripheral portion thereof, with a male screw part 34, which is capable of screw engagement with the female screw part 63 of the first structure 1D.

In the connector 5F, as a result of screw engagement between the female screw part 63 and the male screw part 34, the installed condition is established reliably.

### <Sixth Embodiment>

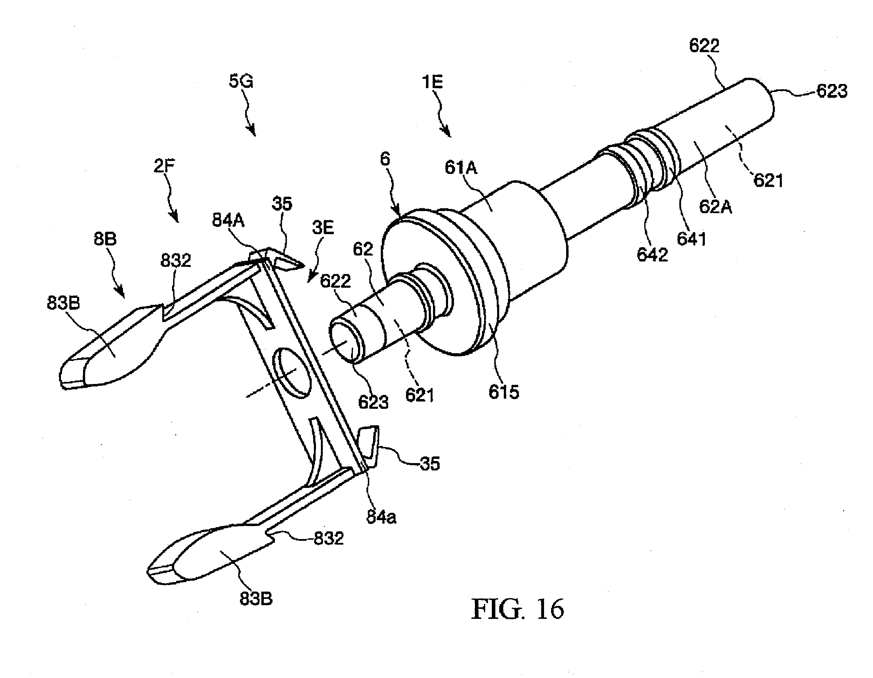

FIG. 16 is an exploded perspective view showing a sixth embodiment of the connector according to the present invention.

Referring to this figure, the sixth embodiment of the connector according to the present invention will be described below. The following description will focus on differences from the above-described embodiments, and descriptions of items, which are the same as those described above, shall be omitted.

The sixth embodiment is the same as the fourth embodiment described above, except that the female connector section and the female lock part are omitted.

In a first structure 1E of a connector 5G, as shown in FIG. 16, the female connector section is omitted, and in place thereof, a tubular part 62A (a second tubular part) is provided, which communicates with a tubular part 62 of a male connector section 6. The tubular part 62A has a luer tapered part 622, the outside diameter of which gradually increases in the distal direction of a male connector section main body 61A, and further has stepped parts 641, 642. The stepped parts 641, 642 are provided mainly for preventing a flexible tube or the like from slipping off, in the case that such a flexible tube or the like is fitted over the tubular part 62A during use thereof. The stepped parts 641, 642 may be omitted, in cases where the fitting strength is sufficient.

In addition, the male connector section main body 61A of the male connector section 6 is formed with a flange part 615.

In a second structure 2F, the female lock section is omitted.

An installation section 3E of the second structure 2F includes a pair of engaging pieces 35, 35, which are capable of engagement with the flange part 615 of the first structure 1E. The engaging pieces 35 are inclined in a direction so as to approach toward each other (toward the inner side). In addition, the engaging pieces 35 are each arranged, respectively, on urging parts 84A.

While the connector according to the present invention has been described above with reference to the embodiments thereof shown in the drawings, the invention is not limited to such embodiments. Each of the components of the connector can be replaced by other arbitrary configurations, which can exhibit functions the same or equivalent to those mentioned above. Additionally, arbitrary components or structures may be added to the aforementioned embodiments.

In addition, according to the present invention, the connector may be formed by a combination of any two or more arbitrary configurations (features) of the above-described embodiments.

Moreover, the mating body to be connected to the connector according to the present invention is not limited to being a connector, such as described in the first embodiment above. Other examples of the mating body may include connectors that concern the interconnection of flows of liquids represented by medical liquids, feeding liquids, etc., for example, connector-fitted tubes of an infusion circuit, a transfusion circuit, an artificial lung circuit, an artificial dialysis circuit, a peritoneal dialysis circuit, a nutrition circuit, etc., catheters that are left together indwelling inside blood vessels, the peritoneum, the nose, intestines, or the like, needles such as indwelling needles, winged needles, etc., prefilled syringes, syringes, and mixture-infusion ports of infusion bags.

### INDUSTRIAL APPLICABILITY

The connector according to the present invention includes a first structure including a tubular male connector section that has a male-side cavity, and a tubular female connector section having a female-side cavity, which communicates with the male-side cavity and to which another male connector section the same as the male connector section can be connected, and a second structure including an installation section that can be installed on the first structure, a male lock section disposed adjacent to the male connector section in an installed condition in which the second structure is installed on the first structure, so that the direction of connection thereof is parallel to that of the male connector section, and a female lock section to which another male lock section the same as the male lock section can be connected, which is disposed adjacent to the female connector section in the installed condition, so that the direction of connection thereof is parallel to that of the female connector section, wherein the first structure can be used selectively with either the second structure installed thereon or without the second structure installed thereon. Therefore, the connector can be appropriately modified in accordance with a mating body to be connected thereto. Specifically, the connector can be used selectively between a condition in which the second structure is installed on the first structure, and a condition in which the second structure is not installed on the first structure. In other words, the form of use of the connector can be changed in accordance with the mating body to be connected to the connector. Consequently, the connector and the mating body can be connected to each other securely, so that flow of a liquid, such as an infusion, in one direction and in another direction through the connector and the mating body can be achieved. Thus, the connector can be used in a variety of ways and is highly versatile. Accordingly, the connector of the present invention has industrial applicability.

## Claims

1. A connector comprising:
a first structure including a tubular male connector section that has a male-side cavity, and a tubular female connector section having a female-side cavity, which communicates with the male-side cavity and to which another male connector section the same as the male connector section can be connected; and
a second structure including an installation section that can be installed on the first structure, a male lock section disposed adjacent to the male connector section in an installed condition of being installed on the first structure, so that the direction of connection thereof is parallel to that of the male connector section, and a female lock section to which another male lock section the same as the male lock section can be connected, which is disposed adjacent to the female connector section in the installed condition, so that the direction of connection thereof is parallel to that of the female connector section;
wherein the first structure can be used selectively with either the second structure installed thereon or without the second structure installed thereon.

2. The connector according to claim 1, wherein the first structure is used with the second structure installed thereon in a case where a mating body using the first structure has the other male connector section and the other male lock section.

3. The connector according to claim 1, wherein the female connector section is formed with a screw part on an outer peripheral portion thereof.

4. The connector according to claim 1, wherein the male connector section is formed with a columnar part, which has a substantially columnar outside shape and a center axis that is substantially orthogonal to a center axis of an opening part of the male connector section; and
the installation section is composed of a recess, which is formed in the vicinity of the male lock section and into which the columnar part can be fitted, and a small plate-like piece having a hole through which the male connector section can pass.

5. The connector according to claim 1, wherein the installation section is composed of a ring-shaped section, into which the outer peripheral portion of the female connector section can be fitted.

6. The connector according to claim 1, wherein the male lock section is provided with a male-side engaging part, and includes a pair of claw parts, which can move toward and away from each other, and an urging part for urging both of the claw parts so as to move the claw parts away from each other; and
the female lock section includes a female-side engaging part capable of engagement with a male-side engaging part of another male lock section, which is the same as that of the male lock section, and an operating part operable to cause both of the claw parts of the male lock section to move closer to each other.

7. The connector according to claim 6, wherein each of the claw parts projects respectively in the same direction as the male connector section in the installed condition.

8. The connector according to claim 6, wherein each of the claw parts projects in a direction opposite to the male connector section in the installed condition.

9. The connector according to claim 1, wherein the female connector section and the male connector section are arranged such that center lines thereof are parallel to each other, and an opening part of the female connector section and an opening part of the male connector section are oriented in opposite directions.

10. The connector according to claim 1, wherein the female connector section and the male connector section are arranged such that center lines thereof are substantially orthogonal to each other.

11. A connector comprising:
a first structure including a tubular male connector section having a male-side cavity; and
a second structure including an installation section that can be installed on the first structure, and a male lock section disposed adjacent to the male connector section in an installed condition in which the second structure is installed on the first structure, so that the direction of connection thereof is parallel to that of the male connector section;
wherein the first structure can be used selectively with either the second structure installed thereon or without the second structure installed thereon.
